# EUROPEAN PATENT APPLICATION

(11) **EP 2 141 246 A1**
(43) Date of publication of application: **06.01.2010**
(21) Application number: 08159400.4
(22) Date of filing: 01.07.2008
(51) Int. Cl.: C12Q 1/68

(54) **Separation-free methods of PCR detection using SERRS**

(71) Applicant: Koninklijke Philips Electronics N.V., 5621 BA Eindhoven (NL)
(72) Inventor: The designation of the inventor has not yet been filed
(74) Representative: Damen, Daniel Martijn

(57) **Abstract**

The present invention relates to methods and devices for sensitive detection of target molecules such as DNA, using surface-enhanced (resonance) Raman spectroscopy (SE(R)RS) in combination with a modified quantitative real-time PCR protocol. The principle of the methods is that the SE(R)RS signal changes in the presence of the target by using SE(R)RS-labelled probes that are degraded during amplification. The methods are sensitive and accurate as well as convenient, as no separation step is needed prior to detection of the SE(R)RS signal.

## Description

### FIELD OF THE INVENTION

The present invention relates to methods and tools for the detection and/or quantification of a nucleic acid analyte using surface-enhanced (resonance) Raman spectroscopy (SE(R)RS). A separation-free, single tube assay is provided, involving a limited number of steps.

### BACKGROUND TO THE INVENTION

The sensitive and accurate detection, either qualitatively or quantitatively, of low concentrations of biomolecules such as proteins, peptides, oligonucleotides, nucleic acids, lipids, polysaccharides, hormones, neurotransmitters, metabolites, etc. has proven to be an elusive goal with widespread potential uses in medical diagnostics, pathology, toxicology, epidemiology, biological warfare, environmental sampling, forensics and numerous other fields such as comparative proteomics and gene expression studies. A particular example is the detection of DNA e.g. in medical diagnostics (the detection of infectious agents like pathogenic bacteria and viruses, the diagnosis of inherited and acquired genetic diseases, etc.), in forensic tests as part of criminal investigations, in paternity disputes, in whole genome sequencing, etc.

Detection of nucleic acids is facilitated by amplifying the nucleic acid to be detected prior to, or during, detection. Typically, this is done by using the polymerase chain reaction (PCR). Instead of merely hybridizing to the nucleotide sequence, the primers used in PCR also serve as a starting point for nucleic acid (e.g. DNA) replication using a polymerase. Detection can be ensured e.g. by hybridizing a labelled probe to the amplified PCR product, or by using labelled primers and detecting the labelled product after removing the unbound primers.

Qualitative PCR is a well established and straightforward technology, but quantification of specific nucleic acids present in a sample is a demanding task. Accurate quantification is hampered by a number of variations that may occur during sample preparation, storage or the course of the reaction. Even minor variations in reaction conditions are greatly magnified by the exponential nature of the PCR amplification. Moreover, the need for a post-amplification step to detect the products may introduce further errors. These variations may partly be overcome by normalising the amount of PCR products of the specific template with respect to an internal reference template.

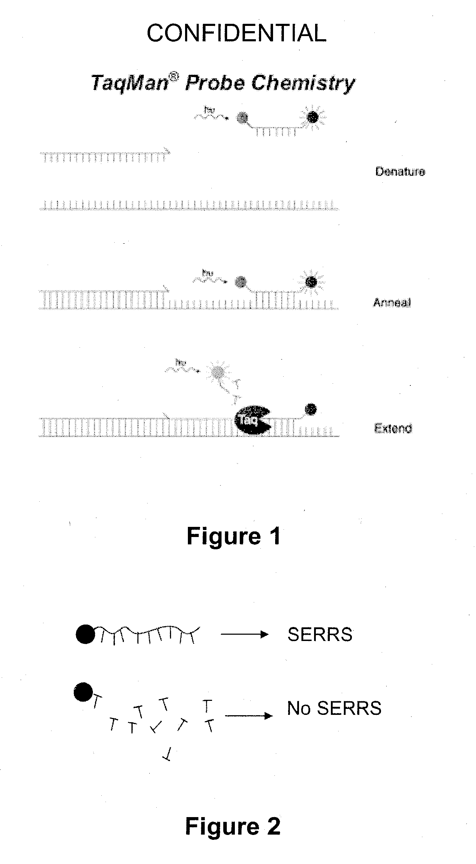

To allow a more accurate quantification, the possibility to monitor the amplification of target nucleic acid in real-time, as well as reduce the number of steps and manipulations needed, new methods have been developed. The most well-known of these real-time, quantitative PCR methods is referred to as TaqMan® PCR, described in US patent No. 5,487,972. Briefly, the PCR is performed using two primers and a (TaqMan®) fluorescent dye-labelled probe located between the two PCR primers. The use of a labelled probe ensures the specific detection of target nucleic acid. The method is based on the 5'-3' exonuclease activity of the Taq DNA polymerase, which results in cleavage of the fluorescently labelled probes during PCR. The intensity of fluorescence can then be measured. The TaqMan® probe is located between the two PCR primers and typically has a melting temperature higher than that of the primers: binding of the fluorescent probe no later than the primers is important because otherwise PCR products would be formed without generation of fluorescence intensity and thus without being detected. The labelled probe has two fluorescent tags attached to it. One is a reporter dye, such as 6-carboxyfluorescein (FAM), of which the emission spectra are quenched due to the spatial proximity of a second fluorescent dye, e.g. 6-carboxy-tetramethyl-rhodamine (TAMRA). During PCR, the Taq polymerase amplifies single stranded DNA molecules starting from the primer. When it encounters a double stranded piece of DNA during the copying process (here on the place where the probe is bound), the 5' → 3' exonuclease activity of the Taq polymerase will ensure that this DNA is degraded. Degradation of the probe by the Taq DNA polymerase frees the reporter dye from the quenching activity of the quenching dye. As a result, the fluorescent activity increases with an increase in cleavage of the probe, which is proportional to the amount of PCR product formed (see Fig. 1). This fluorescent activity can be detected without the need for an extra hybridization or washing step, in the same recipient in which the reaction is performed.

Current detection methods typically involve detection of fluorescently labelled oligonucleotide probes that can bind to the analyte or target nucleic acid. Cross-reactivity and non-specific binding may complicate fluorescent detection of biomolecules in complex samples. Even where high probe-specificity is obtained, the sensitivity of fluorescent detection is often insufficient to identify low concentrations of biomolecules. Whereas the sensitivity may be improved by amplifying the nucleic acid sample prior to or during detection, multiplexing (the simultaneous analysis of multiple signals, e.g. from different targets) remains very difficult. Fluorescence spectra contain broad bands typically in the order of multiple nanometers, therefore multiple fluorescent species in solution need multiple excitation wavelengths to discriminate between the broad band spectra. This considerably hampers the feasibility of working with different quencher and reporter dye pairs.

Moreover, reproducible accurate detection of low concentrations of analytes remains a challenge. Especially for biomolecule detection, amplification or purification is often required to obtain detectable concentrations as biomolecules are often present at very low concentrations. Thus, detection of these molecules often requires (extensive) sample preparation time (e.g. DNA amplification by PCR, followed by removing unbound labels and detecting the signal). For these applications, major challenges are to speed up the sample preparation thereby reducing the analysis time, to make the analysis more sensitive, and more accurate by reducing the number of handling steps. Thus, there is a clear need in the art for methods that are as easy and at least as sensitive as fluorescent detection, better suited to multiplexing, and compatible with PCR amplification, preferably quantitative PCR amplification.

Surface enhanced (resonance) Raman scattering (SE(R)RS) allows detection with very high sensitivity for the measurement of very low concentrations of analyte. Compared to fluorescence a SERS or SERRS signal can be more easily discriminated from contamination and background due to its sharp molecular vibrational bands, instead of broad electronic bands in fluorescence. Under resonant excitation the SERRS signal of molecular vibrations are about 3 orders enhanced with respect to non-resonant excitation in SERS. This further increases specificity of the method.

Another key advantage of SE(R)RS is the possibility of multiplexing using a single excitation wavelength. Indeed, SE(R)RS has the unique feature that the scattered light consists of sharp, molecule-specific vibrational bands which makes discrimination of multiple analytes possible. Each SE(R)RS-active dye used as a label gives a unique fingerprint which can be recognised in a dye mixture without separation as would be necessary for fluorescence spectroscopy. There are many dyes, either specially designed dyes for SE(R)RS or dyes that were previously used in other detection methods, each with a unique spectrum. SE(R)RS is thus a highly sensitive and specific method for biomolecule detection giving increased sensitivity for the detection of low concentrations of biomolecules.

Unlike specific combinations of fluorescent molecules, a SE(R)RS signal of a Raman label is not quenched by the proximity of another SE(R)RS dye. Thus, even when using a probe with both dyes, this would result in a SE(R)RS signal from each of the dyes. Thus, the classical Taqman® PCR can not be combined with SE(R)RS detection.

### SUMMARY OF THE INVENTION

The present invention is based on the finding that when a free Raman dye (or dyes bound to a single nucleotide) is brought into close proximity with a SERS active surface, no or only a very weak SERRS signal is obtained, while when the dye is coupled to a nucleic acid under the same specific set of conditions a strong SERRS signal can be measured. The reasons for this are either because the dye does not adsorb on the SERS active surface or because the distance to the SERS active surface is so small the SE(R)RS signal is quenched.

This opens up possibilities for using SE(R)Rs in real-time PCR, as an alternative to Taqman® PCR. A Raman labelled probe present in a sample potentially containing a target nucleic acid will yield a SE(R)RS signal. When performing a PCR with primers specific for the target nucleic acid using a polymerase having 5'→3' exonuclease activity, the probe will hybridize to the target and be degraded during PCR. Thereby, dye-labelled nucleotides are released. The release of the dye-labelled nucleotides will lead to a decrease in SERRS signal. This decrease in SERRS signal is a direct measure for the amount of target nucleic acid present in the sample. Indeed, when no target nucleic acid is present, the Raman-labelled target-specific probe stays intact and a strong SERRS signal is obtained. (Fig. 2)

A particular advantage of the present findings is that the sensitivity of SE(R)RS can be combined with the advantages of real-time, quantitative PCR detection. This makes it possible to detect a SE(R)RS signal without the need for a hybridization step after amplification, or without the need to remove excess reagents prior to detection. Also, both amplification and detection can be performed in the same reaction container.

As indicated above, particular advantages of using SE(R)RS detection-based methods instead of fluorescence-based methods include the possibility of multiplexing (i.e. the detection of multiple signals at once) due to the sharp, molecule-specific vibrational bands. Also, because of the fact that no quenching is used, the methods can be performed using one Raman label per probe rather than two fluorescent labels. This results in probes that are both cheaper and easier to produce than double-labelled fluorescent probes.

According to one aspect of the invention, methods are provided for detecting one or more target nucleic acids in a sample, which involve using SE(R)RS detection in a real-time PCR. More particularly, methods for detecting one or more target nucleic acids in a sample are provided comprising the steps of:
- adding to the sample:
   one or more pairs of PCR primers each primer pair capable of specifically amplifying one of the one or more target nucleic acids or a part thereof and
- one or more SE(R)RS-labelled nucleic acid probes, each probe capable of specifically hybridizing to one of the one or more target nucleic acids or part thereof that is amplified by the one or more PCR primer pairs;
- performing a PCR reaction using a polymerase with 5'→3' exonuclease activity;
- measuring the SE(R)RS signal of the sample; whereby the SE(R)RS signal in the sample is an indication of the presence of the one or more target nucleic acids.

According to particular embodiments, the polymerase with 5'→3' exonuclease activity is Taq polymerase. However, the use of alternative polymerases is also envisaged.

According to particular embodiments, each of the nucleic acid probes contains one SE(R)RS label, i.e. there is no need for a fluorophore and quencher combination.

According to specific embodiments, the 3' end of the one or more nucleic acid probes is modified to prevent elongation of the probe during PCR. According to further particular embodiments, this modification is achieved by introducing an amine group, a phosphate group, a methyl group or a SE(R)RS label at the 3' end of the probe.

The methods described herein can be performed in real-time, or detection can be done afterwards. According to particular embodiments, the SE(R)RS signal is monitored in real-time. According to alternative embodiments, the SE(R)RS signal is measured as end-point measurement.

According to particular embodiments, the methods also comprise comparing the SE(R)RS signal with that of a control or control sample. According to a specific embodiment, this control sample contains no target nucleic acid, and a decrease in the SERRS signal compared to the control is indicative of the presence of target nucleic acid in the sample. According to an alternative specific embodiment, the control is the SE(R)RS signal of the sample measured after the labelled probes have been added, but prior to performing a PCR reaction, wherein a decrease in the SERRS signal compared to the control is indicative of the presence of target nucleic acid in the sample.

According to particular embodiments, the primers used are capable of amplifying molecules related to the target nucleic acid sequence.

According to particular embodiments, the methods disclosed herein are used for detection of SNPs or particular alleles, or for sequencing.

According to particular embodiments, the SE(R)RS signal is measured in the presence of a SERS-active substrate. According to further particular embodiments, the SE(R)RS-active substrate is a colloidal solution of SERS-active particles. According to yet further particular embodiments, the SERS-active substrate comprises metal particles, most in particular silver particles.

According to further particular embodiments, an aggregating agent is added to the colloidal SERS-active particles prior to measuring the SE(R)RS signal. According to yet further particular embodiments, the aggregating agent is a polyamine, most in particular spermine.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### DESCRIPTION OF THE FIGURES

Fig. 1 shows the general principle of TaqMan® Probe chemistry. The Taqman® probe is an oligonucleotide probe labelled with fluorescent dye on the 5' end and quencher on the 3' end. The close proximity of the dye and quencher results in negligible fluorescence when the probe is intact. However, during polymerisation, the 5'-nuclease activity of Taq polymerase cleaves the probe, separating the dye and quencher, which allows detection of fluorescence. Picture taken from www.bcm.edu
Fig. 2 is a schematic drawing of a dye-labelled SERRS probe when combined with SERRS detection. Top: intact probe, giving rise to a SERRS signal. Bottom: degraded probe with dye-labelled nucleotides, reducing the SERRS signal.

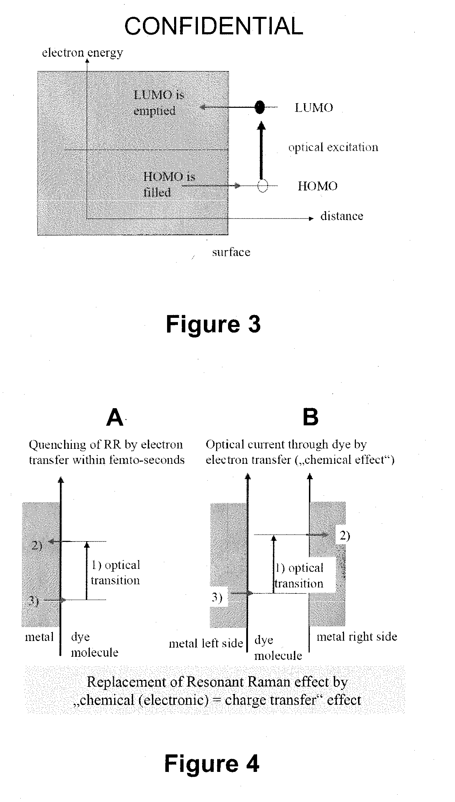

Fig. 3. Quenching of a bare dye molecule adsorbed (in close contact) to a SERS active surface. With distances between dye molecule and surface of less than 0.1 - 0.2 nm the molecular orbitals overlap with the orbitals of the surface (typically metal). In this situation, charge (electron energy, see y axis) can be transferred from the highest occupied molecular orbital (HOMO) via optical (electron resonant) excitation of the dye-molecule to the lowest unoccupied molecular orbital (LUMO). Futamata, Review of "Single Molecule SERS and TERS meeting" in Tsukuba (2006).
Fig. 4. Replacement of resonant Raman (RR) effect by charge transfer effect. The optical excitation effect described in Fig. 3 results in an effective quenching (also called dephasing) of the resonant Raman effect of the molecule by the presence of the SERS active surface. This is shown in panel A. However, when the dye molecule is located in between two metal particles serving as SERS active surface (e.g. a junction of colloidal dimers), the situation shown in panel B, the optical excitation can be used to act as an optical current to transfer electron energy from the metal, via the molecule to the other metal particle, leading to chemical charge transfer effect in SERS. This "chemical or charge transfer" leads to enhancement factors in SE(R)RS on the order of 10-100, which still is much smaller than the electromagnetic enhancement factors 10⁸ or higher, estimated in various SE(R)RS experiments in literature. The resulting SERRS and SERS signals are likely to be comparable for bare dye molecules in very close contact to metal nanoparticles.

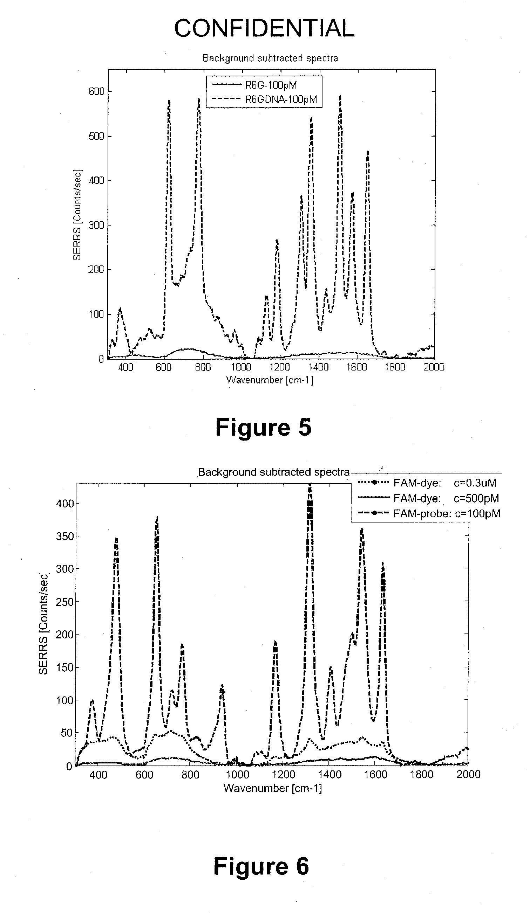

Fig. 5 Comparison of SERRS spectrum of free dye labels and SERRS-labelled probe at a concentration of 100 pM. Shown are background subtracted SERRS spectra from 100 pM R6G covalently coupled to DNA in buffer (blue line), 100 pM R6G (dye only) in buffer (green line). The experimental conditions are described in Example 1. Y-axis: counts/second, X-axis: wavenumber in cm ⁻¹.
Fig. 6. Comparison of SERS spectrum of free dye labels at high concentrations and SERS-labelled probe at a concentration of 100 pM. Experimental conditions are described in Example 2. Shown are background subtracted SERS spectra from 100 pM FAM covalently coupled to DNA (red dashed line), 0.3 µM FAM (dye only) (blue dotted line), 500 pM FAM (dye only) (green solid line). Y-axis: counts/second, X-axis: wavenumber in cm ⁻¹.

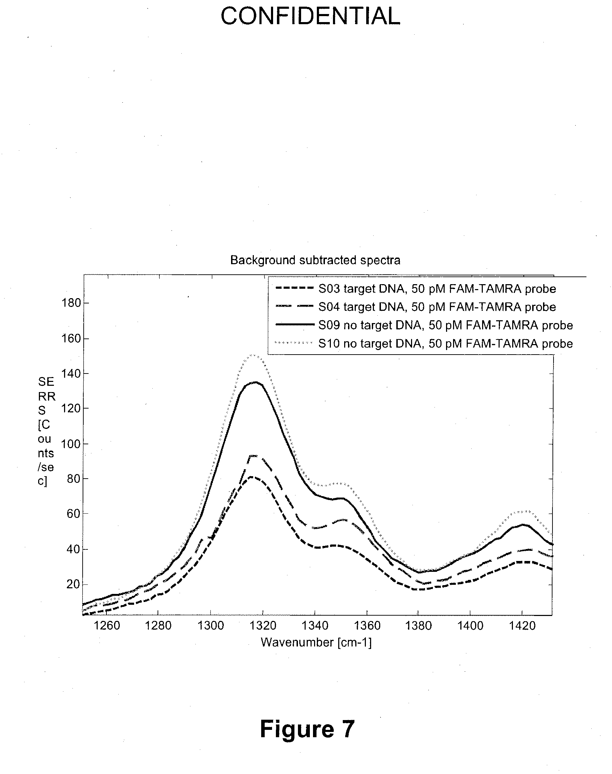

Fig. 7. Comparison of SE(R)RS spectra of non-amplified DNA with SE(R)RS-labelled probes and amplified target DNA with SE(R)RS labelled probes. Experimental conditions are described in Example 3. Background-subtracted spectra are shown for non-amplified DNA with final concentration in SERRS detection of 50 pM double-labelled FAM-TAMRA probe (red solid line and cyan dotted line) and for amplified DNA with 50 pM double-labelled FAM-TAMRA probe (blue short-dashed and green dashed line). Y-axis: counts/second, X-axis: wavenumber in cm ⁻¹.

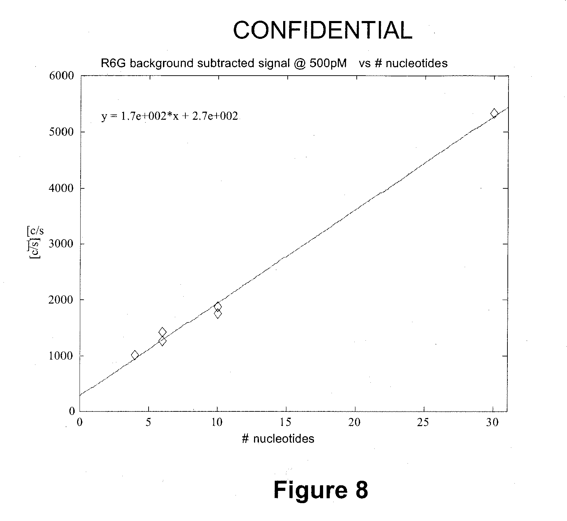

Fig. 8. SERRS signal as a function of number of nucleotides coupled to Rhodamine 6G. Experimental conditions are described in Example 4. Background subtracted signal at 1650cm⁻¹. y-axis: SERRS signal in counts/second, x-axis: number of nucleotides.

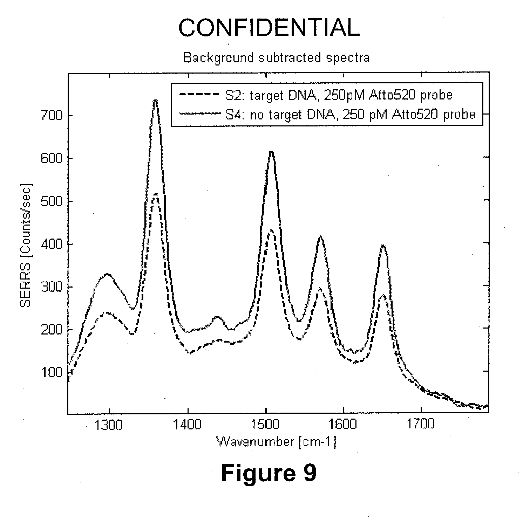

Fig. 9. Comparison of SERRS spectra of non-amplified DNA with SERRS-labelled probes and amplified target DNA with SERRS labelled probes, while using a SERRS probe with a single label. Experimental conditions are described in Example 5. Background-subtracted spectrum is shown for non-amplified DNA with singly labelled Atto520 probe (solid line) and for amplified DNA with singly labelled Atto520 probe (dashed line). Y-axis: counts/second, X-axis: wavenumber in cm⁻¹.

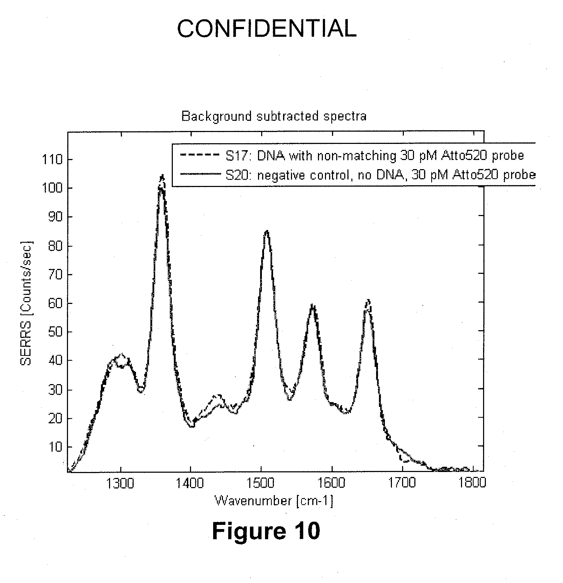

Fig. 10. Comparison of SERRS spectra of a sample containing amplified non-target DNA and a negative control sample (containing primers but no template DNA). A SERRS labelled probe was tested that is not matching the DNA present (single label) to demonstrate that no non-specific signals are obtained. Amplified non-target DNA with singly labelled Atto520 probe (dashed line), negative with singly labelled Atto520 probe (solid line). Y-axis: counts/second, X-axis: wavenumber in cm⁻¹. Signals of two samples are comparable indicating no non-specific reactions. For more details see Example 6.

### DETAILED DESCRIPTION

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The following terms or definitions are provided solely to aid in the understanding of the invention. These definitions should not be construed to have a scope less than understood by a person of ordinary skill in the art.

### DEFINITIONS

The term 'target nucleic acid', as used herein, refers to the nucleic acid to be detected and/or quantified in the methods of the present invention. A non-limiting list of examples of target nucleic acids envisaged in the present application is provided herein below.

The term 'SE(R)RS' as used in the application refers to surface-enhanced (resonance) Raman spectroscopy. A 'Raman label' or `SE(R)RS label' as used throughout the application includes any label, dye or other SERS-active substance capable of generating a SE(R)RS signal when appropriately illuminated. A SE(R)RS label includes both fluorescent and non-fluorescent labels or dyes. A non-limiting list of examples of labels envisaged for use in the methods of the present invention is provided herein below. Typically, the SE(R)RS label will be attached to a (target-specific) probe.

The term 'SE(R)RS spectrum' or 'SE(R)RS signal' refers to the SE(R)RS signal (or spectrum, when the intensity of the signal is measured as function of wavelength) obtained when a SE(R)RS-active substance (such as a SE(R)RS label) is appropriately illuminated or excited. This spectrum can be measured at a specific wavelength or frequency (e.g. a resonance frequency of a SE(R)RS label) or at several or multiple wavelengths (e.g. resonance wavelengths of a dye or several dyes) or across a range of wavelengths, and is specific for the SE(R)RS label. Typically, the incident light is of only one wavelength, while the outgoing (scattered) light is measured across a whole range of wavelengths. However, detection of the scattered light using only one or several wavelengths is also envisaged, as is the use of more than one wavelength for the incident light. Typically, reference will be made to a `SERRS signal' or `SERRS spectrum', which is a signal or spectrum obtained when measuring using the resonance frequency of the dye as excitation.

The term `SERS active surface', as used herein refers to a surface capable of enhancing Raman scattering. Such a surface may be used both for SERS or SERRS detection, but will herein particularly be used for SERRS detection. Typically, a SERS active surface is made of metals, more particularly noble metals such as silver or gold although other materials (copper, platinum, nickel, aluminium, certain alkali metals such as lithium) may be employed as well. The SERS active surface can e.g. be provided as a solid substrate, or in (colloidal) solution in the form of nanoparticles. The solid substrate can take different forms such as a flat sheet, a globular particle, the surface of a container, a tip, or any other shape. The SERS active surface can be the top/outer layer of a structure whereby the nature of the SERS active surface is identical or different from the underlying material (such as e.g. a silver film on a silicone base). Typically, the SERS active surface will however be provided as a homogeneous solution of colloidal nanoparticles.

A 'primer' or 'PCR primer' as used herein is a nucleic acid strand or a related molecule that serves as a starting point for nucleic acid replication. To this end, primers specifically anneal to a target nucleic acid molecule (typically because they have a sequence complementary thereto). The polymerase used for the nucleic acid replication is capable of elongating the primer at its 3' end in a 5'→3' direction. Primers are typically used in pairs, a primer pair capable of specifically amplifying a target nucleic acid or a part thereof will hybridize on sufficient distance from each other on the target nucleic acid to allow a nucleic acid probe to hybridize between the two primers, i.e. in the part of the nucleic acid molecule that is amplified.

The term 'amplified nucleic acid' or 'amplicon' as used herein refers to the product of a nucleic acid amplification reaction. The amplified nucleic acid can correspond to the analyte or a fragment thereof.

The term '(target) probe', 'nucleic acid probe' or 'target-specific probe' as used in the application refers to a molecule capable of specifically binding to (part of) a target nucleic acid molecule. Typically, the specific binding to the target nucleic acid occurs through hybridization of complementary sequences, i.e. the probe is complementary to a part of the target nucleic acid molecule. A `SE(R)RS labelled nucleic acid probe' as used in the application is a probe carrying one or more SE(R)RS labels.

A 'polymerase' is an enzyme capable of the polymerization of new DNA or RNA against an existing DNA or RNA template. During this process, nucleotides are taken from solution, and the synthesis of a polynucleotide sequence against a nucleotide template strand using base-pairing interactions is catalysed. A 'polymerase with 5'→3' exonuclease activity' as used herein refers to a polymerase enzyme which also has another enzymatic activity, namely 5' → 3' exonuclease activity, allowing it to degrade the double-stranded nucleic acid strands which it encounters during the polymerization process. A non-exhaustive list of suitable polymerases is provided herein below.

A 'sample' as used herein relates to a composition potentially comprising at least one target nucleic acid of interest, for which analysis is desired.

A 'control' or 'control sample' as used herein relates to a solution that is identical or very similar in composition to the sample which is generally used to check the accuracy of detection, but for which the presence (and or quantity) of the target nucleic acid is known (positive control: contains target nucleic acid; negative control: does not contain target nucleic acid). The sample itself can also be used as a reference in the methods of the present invention i.e. by measuring the sample before and after performing the methods described herein.

An 'aggregating agent' is a term used in the application to refer to compounds that are capable of promoting the aggregation of nanoparticles. Typical examples of aggregating agents are polyamines, in particular spermine, but other aggregating agents may be used as well (e.g. salts). A non-limiting list of suitable examples of aggregating agents is provided herein below.

The present invention relates to methods and tools for a sensitive detection of a target nucleic acid using surface-enhanced (resonance) Raman spectroscopy (SE(R)RS). Both the presence and optionally the quantity of the target molecule in a sample may be detected. Accordingly, the methods of the invention are typically carried out on a sample containing the target molecule, or suspected of containing the target molecule.

More particularly, methods and tools are provided whereby the target is detected making use of one or more nucleic acid probes which comprise one or more SE(R)RS labels. More particularly, methods are provided which are based on PCR in combination with hybridization of a labelled probe and degradation of the hybridized labelled probe during the PCR amplification. The accompanying change in SE(R)RS signal is indicative of the presence of the target nucleic acid. Using the methods described herein, multiple target nucleic acids can be detected simultaneously, due to the suitability of SE(R)RS for multiplexing.

In methods and tools described herein, PCR primers used are capable of amplifying the target nucleic acid or a sequence thereof. In addition, a SE(R)RS-labelled nucleic acid probe is used which binds to a part of the target nucleic acid located between the two PCR primers. During PCR, the polymerase starting from the primer will encounter the labelled probe hybridized to the target nucleic acid when moving along the target nucleic acid. Thus, the probe typically binds to/is complementary to the amplified nucleic acid or amplicon of the target nucleic acid.

According to particular embodiments of the invention, methods are provided for detecting one or more target nucleic acids in a sample, comprising the steps of:
- adding to the sample:
   - one or more pairs of PCR primers, each primer pair capable of specifically amplifying one of the one or more target nucleic acids or a part thereof, and
   - one or more SE(R)RS-labelled nucleic acid probes, each probe capable of specifically hybridizing to one of the one or more target nucleic acids or part thereof amplified by the one or more PCR primer pairs;
- performing a PCR reaction using a polymerase with 5'→3' exonuclease activity;
- measuring the SE(R)RS signal of the sample, whereby the signal is indicative of the presence and/or amount of the one or more target nucleic acids in the sample.

According to the invention, when the target nucleic acid is present in the sample, both the PCR primers and the labelled probe(s) specific for that sample will hybridize thereto.

According to particular embodiments of the tools and methods of the invention, the target molecule to be detected is a nucleic acid. Examples of nucleic acids include genes, viral RNA, DNA or fragments thereof, bacterial DNA, RNA or fragments thereof, fungal DNA or RNA or fragments thereof, mammalian DNA, cDNA, mRNA, RNA or fragments thereof, peptide nucleic acid (PNA), oligonucleotides, synthetic oligonucleotides, modified oligonucleotides, single-stranded and double-stranded nucleic acids, natural and synthetic nucleic acids, or molecules containing such nucleic acids.

During the PCR reaction, nucleic acid replication of the target nucleic acid is initiated from the primers hybridizing thereto by the nucleic acid polymerase. The nucleic acid polymerase used in methods described herein possesses, apart from its polymerase activity, a 5' to 3' (exo)nuclease activity. This activity implies that the nucleic acid polymerase cleaves double stranded DNA which it encounters, e.g. an oligonucleotide annealed to a complementary nucleic acid. Thus, mononucleotides or small oligonucleotides are released from the probe hybridizing to the target nucleic acid. In order for cleavage by the polymerase to occur efficiently, an upstream oligonucleotide (e.g. a primer) must also be annealed to the same larger polynucleotide. The 3' end of this upstream oligonucleotide (here a PCR primer) provides the initial binding site for the nucleic acid polymerase. Many suitable polymerases having the 5' to 3' (exo)nuclease activity are known in the art and are commercially available. These include, but are not limited to, Thermus aquaticus (Taq) polymerase, Pfu polymerase, Bca polymerase, DNA Polymerase I, E. coli DNA polymerase I, Thermus thermophilus (Tth) DNA polymerase, Bacillus stearothermophilus DNA polymerase, Thermococcus litoralis DNA polymerase, and polymerases derived therefrom but sold under another commercial name. According to specific embodiments, the polymerase used is a template-dependent polymerase. According to further specific embodiments, the polymerase used is a DNA polymerase. According to a further particular embodiment, the polymerase is Taq polymerase.

In methods of the invention, as soon as the bound polymerase encounters the 5' end of the hybridized oligonucleotide (here a SE(R)RS labelled probe), the polymerase cleaves mononucleotides or small oligonucleotides therefrom. As the polymerization continues, the polymerase progressively cleaves mononucleotides or small oligonucleotides from the 5' end of the downstream oligonucleotide. This cleaving continues until the remainder of the hybridized oligonucleotide has been destabilized to the extent that it dissociates from the template molecule (target nucleic acid). The probe (i.e. hybridizing downstream relative to a PCR primer) used in the methods described herein is labelled with a SE(R)RS label. The signal generated by the SE(R)RS label is optimal in the intact probe. Upon cleavage of the probe into nucleotides or smaller oligonucleotides, the signal decreases. In this way, the present invention makes it possible to identify whether or not and to what extent a sample contains the target sequence.

In methods described herein, the labelled probe is directed to a sequence located between the sequences recognized by the two PCR primers. Typically, as with classical TaqMan® PCR, the labelled probe has a melting temperature equal to or higher than that of the primers. Hybridization of the SE(R)RS-labelled probe prior to the start of polymerisation is important, because otherwise PCR products are formed without cleaving of the labelled probe - and thus without a corresponding change in signal, i.e. without the target being detected. Hybridization of the probe prior to (or at the same time as) the hybridization of the primers to the target nucleic acid is typically ensured by a higher melting temperature of the SE(R)RS-labelled probe than that of the primers. According to particular embodiments, the melting temperature of the probe is between 1 and 25°C higher than that of the PCR primers, more particularly between 3 and 20°C higher, even more particularly between 5 and 15°C higher, most particularly about 10°C higher. According to specific embodiments, the melting temperature of the labelled probe is at least 5°C higher than that of the PCR primers. According to further particular embodiments, the primers and the labelled probe have the same melting temperature. The 'melting temperature' or 'Tₘ' is defined herein as the temperature at which half of the nucleic acid strands are in the double-helical state and half are in single-stranded "random-coil" states, and depends on both the length of the molecule and the specific nucleotide sequence composition of that molecule. Note that for single-stranded oligonucleotides, the Tₘ can also be defined, as these will hybridize to the target nucleic acid and thus also form double-stranded nucleic acid molecules. Methods for determining the Tₘ are well-known in the art and include, but are not limited to, the Wallace method, the salt-adjusted method as proposed by Howley, the nearest-neighbour method, and empirical determination. The way in which the melting temperature is calculated is not essential to the invention, but preferably, the same method is used to determine the melting temperatures of each of the primers and probes, so as to obtain a better comparison.

The primers or probes used in the methods and tools described herein include standard oligonucleotides which are designed to bind specifically or hybridize to the target nucleic acid (or, in case of the probes, to the amplification product of the PCR reaction of the methods described herein). The oligonucleotides can be any nucleic acid, typically of less than 30 nucleotides in length, in particular DNA or RNA. Additionally or alternatively, nucleic acid analogues can be used in the primers or probes, such as peptide nucleic acids (PNAs), locked nucleic acid (LNAs), or morpholinos (Karkare and Bhatnagar (2006) Appl. Microbiol. Biotechnol. 71(5):575-86). However, when using a probe consisting (or partially consisting) of nucleic acid analogues, it should be ensured that the labelled probe can be degraded by the 5'→3' exonuclease activity of the polymerase during a PCR reaction. For primers used in the methods described herein, it should be ensured that the polymerase can initiate transcription using the 3' end of the primer as starting point. According to particular embodiments, the primers used do not carry a fluorescent or Raman label. According to further particular embodiments, the primers are not labelled.

Typically, the target-specific probes suitable for use in the methods and tools of the present invention comprise complementary nucleotide sequences, or derivatives or analogues of such binding pairs. These may be used, as long as the (physical or chemical) binding remains specific. For instance, oligonucleotides may be used that are not 100% complementary, but are still capable of hybridizing under suitable stringency conditions (e.g. with one or two mismatches). The same applies for the primers.

Particular embodiments of the invention relate to methods which discriminate between two closely related target molecules. This is for instance the case in SNP detection or detection of mutations, in the discrimination between different alleles, or in the detection of genes from closely related species (e.g. pathogenic or non-pathogenic species), although these examples are not intended to be limiting. It will be understood by the skilled person that in these embodiments, the specificity of the target probe for the target nucleic acid (or amplicon) is essential and non-specific or low-stringency binding with the target nucleic acid should be excluded. For instance, where the methods of the present invention are used for the discrimination between different target nucleic acids, e.g. for the detection of one target nucleic acid carrying a single nucleotide polymorphism (SNP) and another target nucleotides not carrying the SNP, or for the detection of either of these only, hybridization conditions need to be strict and only perfect matches between probe and the amplified nucleic acid can be allowed. Thus, according to particular embodiments, the methods are used for selective discrimination between target nucleic acids (or to very specifically identify the presence of a target, in a sample potentially comprising similar sequences), and the SE(R)RS labelled nucleic acid probe is selected such that it is specific for each/one particular target nucleic acid. According to further embodiments, methods used for selective discrimination between target nucleic acids make use of differentially labelled nucleic acid probes, each specific for one particular target nucleic acid. Using methods according to these embodiments, the relative amounts of each target in a sample may be calculated. Alternatively, the differential detection may serve as control, e.g. when only one target nucleic acid can be present, or when the different target nucleic acids should be present in the same amount.

Although stringent conditions may also be applied for hybridization of the primers, discrimination between two closely related sequences will typically be done at the level of the target probe, e.g. for SNP detection. Thus, according to particular embodiments of methods described herein, the primers may also amplify molecules other than the target nucleic acid, more particularly where the sample contains nucleic acids which are related to the target nucleic acid sequence. The target probe should however be specific for the target nucleic acid sequence only. Examples of these situations include primer pairs specific for a certain gene region, while the labelled probe is specific for one allele (and not all alleles) within the region, or primer pairs which amplify a gene sequence occurring in several species, whereas the labelled probe is specific for the gene sequence as occurring in one of the species only. According to further particular embodiments, more than one differentially labelled specific probe may be used, e.g. to detect the presence of other alleles or species.

In methods described herein, the primers and probes used are further characterized by whether or not they serve as starting point of replication. Indeed, an important difference between primers and target probes used in methods described herein is that the 3' end of the primers should be suitable to serve as a starting point for replication of nucleic acids, whereas no replication should be initiated from the target probes used. To this end, it may be advantageous to modify the 3' end of the nucleic acid probe to prevent elongation. This may be achieved by introducing a SE(R)RS label at the 3' end of the probe. Other modifications that prevent elongation of the probe by polymerase during PCR are known to the skilled person and include, but are not limited to, introducing an amine group, a phosphate group, or a methyl group at the 3' end of the probe, or by introducing a phosphate ester or a phosphoramidite such as 3-(4,4'-dimethoxyltrityloxy)propyl-1-{(2-cyanoethyl)-(N,N-diisopropyl)}-phosphoramidite, 2- {2-(4,4'-dimethoxytrityloxy)-ethylsulfonyl}ethyl-(2-cyanoethyl)-(N,N,-di isopropyl)-phosphoramidite), 3- {(4,4'-Dimethoxytrityloxy)-2,2-dicarboxyethyl}propyl-(2-cyanoethyl)-(N,N-di isopropyl)-phosphoramidite, or an inverted 3'-3' linkage at the 3' hydroxyl group of the oligonucleotide primer, as e.g. described for terminator oligos in US patent No. 6248567.

Methods described herein are based on the detection of a SE(R)RS labelled probe. Methods for generating probes comprising one or more Raman labels for use in the methods described herein are known to the skilled person. These include, e.g., methods whereby oligonucleotides are labelled after synthesis. The optical label can be provided on the oligonucleotide, either as an intercalator or by direct covalent attachment (Faulds et al. (2005) Analyst, 130:1125-1131). Additionally or alternatively, an optical label, such as a SE(R)RS label or a fluorescent label can be chemically added at the 5' end of the nucleic acid, e.g., using the EDAC (1-ethyl-3,3-dimethylaminopropyl carbodiimide) method as described in, e.g., Vo-Dinh et al. (1999) J. Raman Spectro. 30:785-793, and U.S. Patent No. 5,814,516. Further methods of labelling oligonucleotides include the chemical modification of the deoxyribose by replacing a DNA base with a SE(R)RS or fluorescent label. This can be done throughout the sequence, thereby integrating more than one label (Cuppoletti et al. (2005) Bioconjug. Chem. 16(3):528-534). An optical label can also be chemically bound to a number of internal phosphate in the oligonucleotide (Mineno et al. (1993) DNA seq. 4(3):135-141).

Alternatively, Raman labelled probes can be generated by making use of labelled nucleotides (see below). SE(R)RS-active labels that contain reactive groups designed to covalently react with other molecules, such as nucleotides or nucleic acids, are commercially available (e.g., Molecular Probes, Eugene, OR). SE(R)RS-active or fluorescent labels that are covalently attached to nucleotide precursors may be purchased from standard commercial sources (e.g., Roche Molecular Biochemicals, Indianapolis, IN; Promega Corp., Madison, WI; Ambion, Inc., Austin, TX; Amersham Pharmacia Biotech, Piscataway, N.J.). The use of modified nucleotides has successfully been used to synthesize multiply labelled oligonucleotides (Misra et al. (2004) Bioconjug. Chem. 15(3):638-646 and Brumbaugh et al. (1988) PNAS 85:5610-5614).

The SE(R)RS labels may be any label or dye capable of being detected by SERS and/or SERRS methods. These include both fluorescent and non-fluorescent labels. SE(R)RS labels suitable for use in the methods of the present invention include, but are not limited to, ROX, rhodamine 6G, HEX, FAM, TET, Cy3, Cy5, Cy3.5, TAMRA, TRIT (tetramethyl rhodamine isothiol), fluorescein dyes, such as 5- (and 6-) carboxy-4',5'-dichloro-2',7'-dimethoxy fluorescein, 5-carboxy-2',4',5',7'-tetrachlorofluorescein and 5-carboxyfluorescein; rhodamine dyes such as 5- (and 6-) carboxy rhodamine, 6-carboxytetramethyl rhodamine and 6-carboxyrhodamine X, phthalocyanines such as methyl, nitrosyl, sulphonyl and amino phthalocyanines, azo dyes such as those listed in U.S. Pat. No. 6,127,120, N,N-diethyl-4-(5'-azobenzotriazolyl)-phenylamine, 4-(4-Aminophenylazo)phenylarsonic acid monosodium salt, arsenazo I, basic fuchsin, Chicago sky blue, direct red 81, disperse orange 3, HABA (2-(4-hydroxyphenylazo)-benzoic acid), erythrosin B, trypan blue, ponceau S, ponceau SS, 1,5-difluoro-2,4-dinitrobenzene, cresyl violet, aminoacridine, p-dimethylaminoazobenzene, azomethines, cyanines and xanthines such as the methyl, nitro, sulphano and amino derivatives, and succinylfluoresceins. Certain naturally occurring compounds such as amino acids (e.g., arginine, methionine, cysteine), nucleic acid bases (e.g., adenine, or guanine) or chemical derivatives thereof can also generate a Raman signal. These and other Raman-active molecules can be obtained from commercial sources. The skilled artisan will realize that the SE(R)RS labels that can be used in the context of the invention are not limited to those disclosed herein, but may include any known SE(R)RS label that can be attached directly or indirectly to a nucleic acid probe.

It is noted that the choice of the label can be influenced by factors such as the resonance frequency of the label, the resonance frequency of other molecules present in the sample, etc... SE(R)RS-active labels of use for detecting biomolecules are described in the art such as in U.S. Pat. Nos. U.S. 5,306,403; U.S. 6,002,471; and U.S. 6,174,677.

Methods of the present invention involve the detection of one or more SE(R)RS labels. Factors that will affect detection are the proximity of the labels to each other and their accessibility for detection. Moreover, the proximity of the labels to the SERS active surface will influence detection. Indeed, it was found that, when operating at the resonance frequency of the SERRS dye, labels attached to nucleotide sequences will show resonance Raman effect, while bare dyes (i.e. SERRS labels not attached to nucleic acids, or only to a few, in particular less than 10, nucleotides) show only surface-enhanced Raman effect, without additional resonance enhancement. Thus, no SERRS signal is obtained for free SERRS dyes using spermine. This is likely because the bare SERRS label gets in very close contact with the SERS active surface, so that the resonant Raman contribution is quenched. SERRS labels attached to a nucleic acid molecule are apparently prevented from getting in very close contact with the surface, and thus the resonant Raman contribution is not quenched.

Detection by surface-enhanced spectroscopies such as surface-enhanced (resonance) Raman spectroscopy (SE(R)RS) is based on the strong enhancement of Raman scattering observed for analytes adsorbed onto an appropriate "SERS-active surface". According to the methods described herein, the SE(R)RS labels (either as SE(R)RS labelled probes or as free SE(R)RS labels) are contacted with a SERS active surface before their SE(R)RS spectrum is measured. Typically, the surface is a noble (Au, Ag, Cu) or alkali (Li, Na, K) metal surface. The metal surface may for instance be an etched or otherwise roughened metallic surface.

According to particular embodiments, the SERS active surface is provided in particulate form (specifically in solution, e.g. a colloidal suspension). Materials that may be used for the nanoparticles (or their surface) include, but are not limited to, polymers, silicon, and metals (naked metal or a metal oxide layer on a metal surface). According to a particular embodiment the nanoparticles are of a noble metal such as gold, silver, platinum, copper or combinations or alloys thereof which are provided as colloids. More particularly, the SERS active surface consists of nanoparticles of silver.

In particular embodiments of the methods of the present invention the SE(R)RS signal is further enhanced by the treatment of the metal surface or nanoparticles with chemicals such as sodium chloride and lithium chloride.

The nanoparticles used in particular embodiments of the methods of the invention can be of any size so long as they give rise to a SE(R)RS effect. Typically they have a diameter of about 1-100 nm, more particularly of 4 - 50 nm, most particularly between 25 - 40 nm. When metal particles are used, the size will vary depending on the type of metal. Methods for obtaining metal particles having a diameter of 40 nm have been described such as in Munro et al. (Munro et al., Langmuir, 1995, 11: 3712-3720). According to particular embodiments, the colloidal nanoparticles are reduced prior to use. This may be achieved using EDTA or citrate-based protocols, as known to those of skill in the art. Alternatively, commercially available reduced colloids may be used.

Typically the particles comprise a core and a surface. They may include an organic coating such as of citrate or of a suitable polymer, such as polylysine or polyphenol, to increase their sorptive capacity. The material of the core of the particles need not necessarily be identical to the material of the nanoparticle surface. Optionally, the nanoparticles may be hollow. According to a particular embodiment, however, the labelled nanoparticles are solid particles entirely made of the same material. The surface of the particle should provide a SERS active surface, to allow SE(R)RS signals to be measured.

The shape of the nanoparticles is not critical for practising the methods of the invention, for instance spherical as well as needle-like particles may be used. According to a particular embodiment, spherical nanoparticles are used.

The particles comprising a SERS-active surface are typically colloidal nanoparticles which are generated in a controlled manner so as to be of a uniform and desired size and shape and as stable as possible against self-aggregation. Processes for preparing such colloids are well known (e.g. described in US Application No. 20050130163). Alternative methods of preparing nanoparticles are known (e.g. U.S. Pat. Nos. 6054495, 6127120, 6149868). Nanoparticles may also be obtained from commercial sources (e.g. Nanoprobes Inc., Yaphank, N.Y.; Polysciences, Inc., Warrington, Pa.).

When nanoparticles are used as SERS active surface in the methods described herein, they typically will be aggregated prior to the actual detection or measuring step.

According to a particular embodiment, aggregation is ensured by adding an aggregating agent prior to detection. This aggregating agent may be provided together with the nanoparticles with the SERS active surface, or before or after the nanoparticles with the SERS active surface. Aggregating agents are compounds that allow the nanoparticles to aggregate in complexes. The use of aggregating agents is well known in the art, and the nature of the aggregating agent is not limiting to the invention, although it is possible that some aggregating agents will allow a more reproducible sensitive detection than others. Typically, aggregation of the nanoparticles is achieved by addition of a polyamine. Suitable polyamines include monomeric or polymeric polyamines, such as spermine or spermidine, 1,4-diaminopiperazine, diethylenetriamine, N-(2-aminoethyl)-1,3-propanediamine, triethylenetetramine and tetraethylenepentamine. According to a most particular embodiment, the aggregation agent is spermine.

The present invention relates to improved methods for the detection and/or quantification of an analyte, i.e. a target nucleic acid, either in pure form or as an analyte in a sample. While the methods described herein will generally refer to 'a target nucleic acid' it is equally envisaged that the methods of the present invention can be applied where several nucleic acids are being detected or quantified simultaneously, using different analyte-specific labels, such as differentially labelled probes. Indeed, one of the advantages of SE(R)RS is that it allows the simultaneous detection of different labels, allowing different targets to be detected simultaneously, often referred to as "multiplexing". In this regard it will be understood by the skilled person that the methods of the present invention can be applied to the detection of different target nucleic acids within one sample, whereby each of the target nucleic acids is detected with multiple probes with the same (and/or different) optical properties.

The present methods allow for the improvement of the sensitivity and accuracy of the detection and/or quantification of one or more target nucleic acids in a sample. The term "sample" is used in a broad sense herein and is intended to include a wide range of biological materials as well as compositions derived or extracted from such biological materials. The sample may be any suitable preparation in which the target nucleic acid is to be detected. The sample may comprise, for instance, a body tissue or fluid such as but not limited to blood (including plasma and platelet fractions), spinal fluid, mucus, sputum, saliva, semen, stool or urine or any fraction thereof. Exemplary samples include whole blood, red blood cells, white blood cells, buffy coat, hair, nails and cuticle material, swabs, including but not limited to buccal swabs, throat swabs, vaginal swabs, urethral swabs, cervical swabs, rectal swabs, lesion swabs, abscess swabs, nasopharyngeal swabs, nasal swabs and the like, lymphatic fluid, amniotic fluid, cerebrospinal fluid, peritoneal effusions, pleural effusions, fluid from cysts, synovial fluid, vitreous humor, aqueous humor, bursa fluid, eye washes, eye aspirates, plasma, serum, pulmonary lavage, lung aspirates, biopsy material of any tissue in the body. The skilled artisan will appreciate that lysates, extracts, or material obtained from any of the above exemplary biological samples are also considered as samples. Tissue culture cells, including explanted material, primary cells, secondary cell lines, and the like, as well as lysates, extracts, supernatants or materials obtained from any cells, tissues or organs, are also within the meaning of the term biological sample as used herein. Samples comprising microorganisms and viruses are also envisaged in the context of analyte detection using the methods of the invention. Materials obtained from forensic settings are also within the intended meaning of the term sample. Samples may also comprise foodstuffs and beverages, water suspected of contamination, etc. These lists are not intended to be exhaustive.

In particular embodiments of the invention, the sample is pre-treated to facilitate the detection of the sample with the detection method. For instance, typically a pre-treatment of the sample resulting in a semi-isolation or isolation of the target nucleic acid or ensuring the amplification of the target nucleic acid is envisaged. Many methods and kits are available for pre-treating samples of various types. However, according to particular embodiments, no or only a limited pre-treatment is performed on the sample. According to these embodiments, pre-treatment of the sample does not involve an amplification step of the target nucleic acid, as the detection itself involves amplification of target nucleic acid. In particular when quantification of the target molecule is envisaged, no pre-amplification will take place.

Particular embodiments of the methods provided herein relate to methods of detection based on SE(R)RS, and optionally involve operating at the resonant frequency of SERRS labels used, which gives increased sensitivity. The light source used to generate the Raman spectrum for this purpose is a coherent light source, e.g., a laser, tuned substantially to the maximum absorption frequency of the label being used. This frequency may shift slightly on association of the label with the SERS-active surface and based on the binding to or incorporation in the amplicon, but the skilled person will be well able to tune the light source to accommodate this. The light source may be tuned to a frequency in the absorption band, or near to the label's absorption maximum, or to a frequency at or near that of a secondary peak in the label's absorption spectrum. SERRS detection may alternatively involve operating at, or close to, the resonant frequency of the plasmons on the active surface of the metal colloids. As stated above, the choice of the optimal operating frequency can easily be made by one of ordinary skill in the art based on the metal(s) and label(s) chosen.

In SE(R)RS detection typically the fingerprint spectrum is measured in order to identify each label. However, if the different labels used each have a unique spectral line, then it can be sufficient to detect signal intensity at a chosen spectral line frequency or frequencies.

Typically, the detection step in the SE(R)RS based detection methods of the present invention is carried out using incident light from a laser, having a frequency in the visible spectrum. The exact frequency chosen will depend on the label, surface and analyte. Frequencies in the red and/or green area of the visible spectrum tend, on the whole, to give rise to better surface enhancement effects. However, it is possible to envisage situations in which other frequencies, for instance in the ultraviolet, the near-infrared or infrared ranges, might be used. The selection and, if necessary, tuning of an appropriate light source, with an appropriate frequency and power, will be well within the capabilities of one of ordinary skill in the art, particularly on referring to the available SE(R)RS literature.

Excitation sources for use in SE(R)RS-based detection methods include, but are not limited to, nitrogen lasers, helium-cadmium lasers, argon ion lasers, krypton ion lasers, etc... Multiple lasers can provide a wide choice of excitation lines which is critical for resonance Raman spectroscopy. The laser power, or excitation power, can be varied depending, e.g., on the properties of the label used.

The excitation beam may be spectrally purified with a bandpass filter and may be focused on a substrate using an objective lens. The objective lens may be used to both excite the sample and to collect the Raman signal, by using a holographic beam splitter to produce a right-angle geometry for the excitation beam and the emitted Raman signal. The intensity of the Raman signal needs to be measured against an intense background from the excitation beam. The background is primarily Rayleigh scattered light and specular reflection, which can be selectively removed with high efficiency optical filters. For example, a holographic notch filter may be used to reduce Rayleigh scattered radiation.

Several objective lenses can be used in the context of the invention. According to a specific embodiment, the objective lens is a 5x, 10x, 20x, 50x or a 100x objective lens. In most set ups, the nature of the objective lens influences the excitation volume, since the focal distance, numerical aperture and working distance determine the volume irradiated. Accordingly, where objectives of 5x, 10x, and 50x are used, the excitation volume typically decreases from 5x to 50x, accordingly.

Detection of the SE(R)RS spectrum of one or more SE(R)RS labels as envisaged in methods described herein may be done using devices known in the art, e.g. commercially available spectrophotometers. The surface-enhanced Raman emission signal may be detected by a Raman detector. A variety of detection units of potential use in Raman spectroscopy are known in the art and any known Raman detection unit may be used. An example of a Raman detection unit is disclosed e.g. in U.S. Pat. No. 6,002,471. Other types of detectors may be used, such as a charge-coupled device (CCD), with a red-enhanced intensified charge-coupled device (RE-ICCD), a silicon photodiode, or photomultiplier tubes arranged either singly or in series for cascade amplification of the signal. Photon counting electronics can be used for sensitive detection. The choice of detector will largely depend on the sensitivity of detection required to carry out a particular assay. Several devices are suitable for collecting SE(R)RS signals, including wavelength selective mirrors, holographic optical elements for scattered light detection and fibre-optic waveguides. Various options are discussed, e.g. in WO 97/05280.

The apparatus used for obtaining and/or analysing a SE(R)RS spectrum as envisaged in methods according to the invention may include some form of data processor such as a computer. Once the SE(R)RS signal has been captured by an appropriate detector, its frequency and intensity data will typically be passed on to a computer for analysis. Either the fingerprint Raman spectrum will be compared to reference spectra for identification of the detected Raman active compound or the signal intensity at the measured frequencies will be used to calculate the amount of Raman active compound detected. Therefore, the detection can be qualitative, semi-quantitative or quantitative, when the various spectra obtained as described are compared with an internal standard chosen appropriately, as is well known by a person skilled in the art.

According to the methods provided herein, the measurement of the SE(R)RS spectrum of the SE(R)RS labels is an indication of the presence and/or quantity of a target in a sample. Indeed, the intensity and the nature of the SE(R)RS spectrum detected can be correlated to the presence of a target molecule in the sample. According to particular embodiments, the methods comprise an additional step, wherein the SE(R)RS spectrum or the intensity of the SE(R)RS spectrum is correlated to the presence of the target molecule. When using quantitative correlation, a calibration curve can be made first, to assess the nature of the correlation between SE(R)RS signal and amount of target molecule.

Moreover, methods according to the invention may involve detection of the SE(R)RS spectrum prior to and after PCR amplification. Prior to PCR, the signal of the probe is detectable in the sample. Depending on the presence of target nucleic acid (and the amount thereof), the signal will decrease during PCR. Accordingly methods described herein may involve detection prior to PCR (after addition of at least the target probe) and after PCR amplification and optionally, during PCR amplification and determining the decrease in SE(R)RS signal.

Particular embodiments of the methods further envisage the detection of controls or internal standards, which allow the identification of false positives or negatives and allow for standardisation. A 'positive control' as used herein is a control wherein the target nucleic acid is present. Accordingly, a positive control will give rise to a SERRS signal lower than before PCR. Labelled probes are degraded as a result of hybridization with the target nucleic acid and degradation by the polymerase (assuming that the other reagents are not limiting the PCR reaction). A 'negative control' is a control which does not contain the target nucleic acid. A negative control gives rise to the maximal SERRS signal when measured under the same conditions as the sample. According to further particular embodiments, part of the sample is used to serve as control, while the other part is used for detection. According to particular embodiments, the negative control is a control sample where all constituents of the sample are present in comparable amounts, except for the target nucleic acid, and to which the same amount of SE(R)RS labelled probes has been added. The SE(R)RS signal of this control can be measured either before, after or during the PCR reaction.

A decrease in SE(R)RS signal (or a lower signal) compared to a negative control (i.e. maximal signal) is indicative of the presence of the target nucleic acid. The decrease can be quantitatively correlated to the amount of target nucleic acid present in the sample. More particularly, when quantitative real-time PCR is used, the decrease can be correlated to the amount of target nucleic acid originally present in the sample, prior to amplification.

An important difference of the methods provided herein and the classical, fluorescence-based Taqman® technology, is that in the present methods only one SE(R)RS label is necessary per probe. This is not possible using fluorescence, as the quenching by a second label is necessary to distinguish between unbound probe and bound and degraded probe. Using a SE(R)RS-based method for detection, there is no need for both a fluorophore and an additional quencher, as the change in the SE(R)RS signal is based on the length of the oligonucleotide attached to the label. This has several advantages, as probes carrying only one label are easier and cheaper to manufacture than probes carrying two labels, especially at specified positions. Moreover, as only one label is needed per probe, this significantly increases the amount of labels available for multiplexing applications. As a result, more different specific targets may be detected simultaneously in one measurement. According to particular embodiments, only one SE(R)RS label is used per nucleic acid probe. According to alternative embodiments, more than one SE(R)RS label is used per nucleic acid probe, but none of these labels is a fluorescent quencher, in particular none of these labels is a quencher for another label used on the probe. According to specific embodiments, the use of one or more SE(R)RS labels that are not fluorescent labels is envisaged.

In particular embodiments of methods described herein, the use of probes with more than one SE(R)RS label is envisaged. Such multiple probes are not used to quench the signal, but rather to increase sensitivity and/or specificity of the assays. Of note, the methods presented herein already offer increased specificity compared to traditional PCR assays due to the use of both at least one primer pair and at least one probe (i.e. at least three oligonucleotides which need to hybridize to the target nucleic acid).

When a probe carrying more than one different SE(R)RS label is used, this gives rise to different SE(R)RS spectra (or a SE(R)RS spectrum from different components). This allows the (qualitative or quantitative) detection of one target molecule through at least the combination of two different SE(R)RS spectra. In this way, a "double" detection is ensured, improving accuracy of detection. This is generally achieved by directly or indirectly labelling (each of) the analyte(s) with two or more labels having different optical properties, such that, in parallel, different optical signals are generated, each representative of the presence and/or quantity of the analyte in the sample.

This can be ensured by making use of one probe comprising two or more labels with different optical properties. According to alternative embodiments however, this is ensured by contacting the analyte with two or more probes each carrying a different optical label. As the analyte of interest is a nucleic acid molecule, the labelled probes are each directed against a different sequence within the target nucleic acid, such that the different probes can bind to the target molecule simultaneously. The labelled probes can be selected such that the different signals generated upon the binding of the different probes to the nucleic acid do not interfere.

The at least two SE(R)RS labels with different optical properties may be present in equal amounts, if each probe carries the same labels. Alternatively, the SE(R)RS labels are present in different amounts (e.g. 90% of one SE(R)RS label, 10% of another), and the respective strength of the signals may be used for standardisation purposes. The latter may, in addition to the methods described above, also be achieved by using a mixture of single and double labelled probes.

Alternatively, if the probe is labelled with more than one identical label, the generated signal will be higher, resulting in an enlarged window of detection, i.e. an increased difference between positive and negative control. It should be understood that both embodiments can be combined: a labelled probe may contain more than one identical label to increase signal strength as well as an additional different Raman label to increase specificity.

Additionally or alternatively, detection of more than one SE(R)RS label is envisaged in multiplexing applications. Where methods of the invention are used in multiplex assays (i.e. to detect more than one target nucleic acid molecule simultaneously), a different SE(R)RS label is used for each target and appropriate target-specific probes are used. The intensity of each of the SE(R)RS spectra is then correlated with the presence of the corresponding target nucleic acid molecule in the sample. Typically this implies the use of differentially detectable SE(R)RS labels for each target, though it is envisaged that for particular purposes, i.e. where the contribution of each target is not critical, that identical labels are used. In any case this implies the use of appropriate target-specific probes for each target nucleic acid molecule to be detected. If the target nucleic acids are closely related (e.g. in the case of SNP detection), it may be possible that one primer pair can be used to amplify different target nucleic acids or parts thereof. Typically, however, different primer pairs will be used to amplify different target nucleic acids.

Using methods according to these embodiments, the relative amounts of target nucleic acid present may be determined. Alternatively, the differential detection may serve as control, e.g. when only one target nucleic acid can be present, or when the target nucleic acids should be present in the same amount.

According to a particular embodiment, excitation is done at a single wavelength. The SE(R)RS signal can then be detected at a single wavelength. Alternatively, the whole SE(R)RS spectrum can be detected. A single spectrum is measured of the mixture of SE(R)RS labels, but it consists of contributions of the different labels, and specific peaks belong to specific labels. According to another embodiment, excitation can be done at more than one wavelength. This means that multiple measurements need to be taken. For every excitation wavelength, detection may be done at a single wavelength (detection of a SE(R)RS signal) or for the whole spectrum (detection of a SE(R)RS spectrum). The performing of multiple measurements (using several excitation wavelengths) may ensure that each distinct label is detected properly. According to a particular embodiment, the SE(R)RS signal of each of the SE(R)RS labels is detected at the wavelength which is optimal for that SE(R)RS label, i.e. a SERRS measurement is performed, at the resonance wavelength of the SERRS label. Alternatively, multiple excitation wavelengths can be used and detection can be performed at a single wavelength or multiple wavelengths, or the whole spectrum is detected.

According to methods of the present invention, the measurement of the SE(R)RS spectrum of the SE(R)RS labels is an indication of the presence and/or quantity of a target in a sample. Indeed, the intensity and the nature of the SE(R)RS spectrum detected can be correlated to the presence of one (or different) target molecule(s) in the sample. When using quantitative correlation, a calibration curve can be made first, to assess the nature of the correlation between SE(R)RS signal and amount of target molecule. Of note, in the methods described herein, the intensity of the SE(R)RS spectrum and the amount of the target nucleic acid are correlated, as the SE(R)RS signal will change during the amplification of the target nucleic acid. In particular embodiments, the intensity of the SE(R)RS spectrum and the amount of the target nucleic acid are inversely correlated, as the SE(R)RS signal decreases during the amplification of the target nucleic acid.

For the embodiments of the methods described above involving detection making use of a SERS active surface which is provided by (nano)particles in suspension, the detection can be carried out completely in solution. Detection of the SE(R)RS labels in solution has several advantages over detection on a solid surface. It has been found that the generated signal is stronger. Another advantage is that target binding is faster in solution, i.e. the kinetics of a homogeneous assay are superior, resulting in increased sensitivity and decreased assay time. Although a solid SERS active surface may also be used in the methods described herein, this is typically more cumbersome, as the SE(R)RS labels need to adsorb (actively or passively) to a SERS active surface for detection of their SE(R)RS spectrum. This is more time-consuming for a solid surface than when using a colloidal solution, which also makes it harder to combine accurate SE(R)RS measurements with a real-time PCR.

According to particular embodiments, the concentration of the labelled probes is in the same order of magnitude as the expected concentration of (amplified) analyte, to ensure that the decrease of the SERRS signal can be reliably measured - i.e. it will be ensured that the excess amount of probe does not interfere with analyte detection. According to particular embodiments, the labelled probe is added in a concentration not exceeding the expected concentration of the amplified analyte.

Moreover, the methods presented herein are very suitable to combine with real-time detection. The detectable signal changes with each cycle of PCR amplification. Real-time detection offers the possibility of acquiring data when PCR is still in the exponential phase, which is more reliable than traditional end-point measurements, as these are more prone to be influenced by other factors (e.g. exhaustion of reagents, exponential amplification of earlier mismatches). According to particular embodiments, the SE(R)RS signal of the sample is measured (monitored) in real-time. This may further involve quantification of the sample, e.g. via determining of a threshold cycle to estimate the original copy number of the target nucleic acid, as is known in the state of the art. According to particular embodiments, the SE(R)RS signal is measured as end-point measurement.

Furthermore, it was found that the presence of a solution of colloidal nanoparticles does not interfere with the PCR reaction. Also, SERRS detection can be performed in real time. According to alternative embodiments, SE(R)RS detection is performed as end-point measurement. The methods presented herein are particularly well suited for detection of very low concentrations of analytes. Accordingly, in specific embodiments of the methods provided herein, the concentration of labelled probes added to the sample is low, in particular < 1 µM, more in particular < 500 nM, even more in particular < 100 nM, even more in particular < 50 nM, yet even more in particular < 10 nM, most in particular < 1 nM.

The methods provided herein allow sensitive and accurate detection, either qualitatively or quantitatively, of low concentrations of nucleic acids. These methods have widespread potential uses in medical diagnostics, pathology, toxicology, epidemiology, infectious diseases, biodefense, biological warfare, environmental sampling, food sampling, forensics and numerous other fields such as gene expression studies. Particular examples include, but are not limited to, the detection of DNA in, e.g., medical diagnostics (detection of infectious agents such as pathogenic bacteria and viruses, diagnosis of inherited and acquired genetic diseases, etc...), in forensic tests as part of criminal investigations, in paternity disputes, or in whole genome sequencing.

### EXAMPLES

### Example 1. Comparison between SERRS spectra of DNA-bound and free SERRS dye labels.

SERRS signals were measured for both free dye labels and dye labels covalently coupled to a DNA oligonucleotide probe. To this end, 100 pM R6G dye covalently coupled to DNA (length oligonucleotide: 30 nucleotides) and 100 pM free R6G label were measured. Briefly, the dye (or dye-labelled probe) was added to a colloidal solution of EDTA reduced silver nanoparticles (final concentration in the measurement: 23%, prepared according to Heard et al. Jounal of Colloid and Interface Science Vol 93 (1983), 535) in a buffer. The nanoparticles were aggregated using spermine (final concentration in the measurements 0.1mM), after which the SERRS spectrum was measured using an excitation wavelength of 532 nm (Raman Systems Inc). The background subtracted spectra are shown in Fig. 5. It can be observed that the bare dye (continuous line) does not yield a SERRS spectrum, whereas the dye coupled to DNA (dashed line) gives rise to characteristic SERRS peaks.

Without being bound to the particular mechanism underlying these observations, a possible explanation for this effect could be as follows. Consider a free/bare dye molecule adsorbed (in close contact) to a SERS active surface.. With distances between dye molecule and metal (or in general SERS active) surface of less than 0.1-0.2 nm the molecular orbitals overlap with the orbitals of the metal. In this situation, charge (electron energy) can be transferred from the highest occupied molecular orbital (HOMO) via optical (electron resonant) excitation of the dye-molecule to the lowest unoccupied molecular orbital (LUMO). See Fig. 3. This results in an effective quenching (also called dephasing) of the resonant Raman effect of the molecule by the presence of the metal particle (Fig. 4). This dephasing happens on a time-scale of ∼ 10 fs (10⁻¹⁴s).

However, when the dye molecule is located in between two metal particles (e.g. a junction of colloidal dimers), the optical excitation can be used to act as an optical current to transfer electron energy from the metal, via the molecule to the other metal particle, leading to chemical charge transfer effect in SERS. This "chemical or charge transfer" leads to enhancement factors in SE(R)RS on the order of 10-100, which still is much smaller than the electromagnetic enhancement factors of 10⁸ or higher, estimated in various SE(R)RS experiments in literature. The generally accepted fact is that SERRS gets highest contribution from molecules in between junctions (also called hot-spots, ideally dimers) with a distance between them on the order of 1 nm for highest electrical field enhancements.

Normally resonance Raman lifetimes in the excited state of the dye molecules free in solution (not in close contact to metal particles) are on the order of tens of picoseconds - nanoseconds (roughly 10⁻¹¹ s - 10⁻⁹ s). Although the lifetime of excited molecules near small metal spheres decrease as a result of increase in the rate of non-radiative transfer of energy into the metal, the lifetimes are much longer than the typical lifetimes of the collective electron oscillations (i.e. plasmons) in the metal which are on the order of 10 fs. It is therefore assumed that electromagnetic coupling of the plasmons with the resonantly excited molecules is not as effective when the dye molecules are in very close contact with the SERS active surface, due to dephasing and due to longer lifetimes of resonant Raman as described above.

Under non-resonant excitation, the electron is not excited and the quenching does not take place. Also the non-resonant Raman effect takes place almost instantaneously, on a much faster timescale (- 10⁻¹⁸ s) compared to resonant excitation, which enables multiple non-resonant Raman processes to couple to the plasmons during the lifetime of the plasmons. Therefore, the resulting SERRS and SERS signals are likely to be comparable for bare dye molecules in very close contact to metal nanoparticles.

For SERRS on probes consisting of a dye molecule attached to a nucleic acid strand, it is considered that due to the negative charge of the nucleic acid (in particular DNA) strand the dye molecule is hampered to get in very close contact with the SERS active surface (e.g. a metal particle). As a consequence of this shielding of the nanoparticles, the dye is not in very close contact with the SERS active surface and the resonant Raman contribution is not quenched. Highly likely, the distance between dye and SERS active surface is too large (i.e. more than 0.2 nm) for dephasing to occur.

It should be stressed that, although this is a possible explanation for the observed effect, the invention is not limited by this particular theory, as the methods described herein can be practised irrespective of the underlying mechanism.

### Example 2. Comparison between SERRS spectra of low concentrations of DNA-bound SERRS dye labels and high concentrations of free SERRS dye labels.

It was further determined whether the observed difference in SERRS signalling was concentration-dependent. To see whether the absence of SERRS signal from bare dye was not solely due to the relatively low concentration used for detection, higher concentrations dye were also tested. The SERRS signal of a FAM label covalently coupled to a DNA probe (concentration: 100 pM) was compared to the SERRS signal of free FAM dye at 500 pM (concentration 5x higher) and FAM bare dye at a concentration of 0.3 µM (concentration 3000x higher). The experimental set-up and conditions were the same as described for the R6G experiment in Example 1. As can be seen in Fig. 6, a clear SERRS spectrum is obtained using the concentration of 100 pM FAM label covalently coupled to DNA (dashed line). Using a 5x higher concentration of free dye yielded no detectable SERRS spectrum (continuous line). When the free FAM label was 3000x more concentrated (dotted line) the spectrum was still negligible compared to that observed for DNA-coupled FAM dye at much lower concentrations.

The observation that very high concentrations of FAM bare dye do yield a weak SERRS spectrum can likely be ascribed to the more off-resonance excitation condition for FAM using the 532 nm excitation wavelength. Nevertheless, it is clear that, when using comparable concentrations, no SERRS spectrum is observed for bare dye when compared to dye-labelled probe. Moreover, it should be noted that using the methods presented herein, the total concentration of label remains constant, and the concentration of free SERRS label can thus never rise above the concentration of probe-bound labels originally added to the sample.

### Example 3. Measuring SERRS spectra after performing PCR on sample DNA.

Four samples were tested, two did contain target DNA (S3, S4), two did not (S9, S10). To all samples 100 nM PCR primers were added. To each sample, 25 nM of a specific double-labelled 29-bases probe was added The SERRS labelled probe carries a FAM label at its 5' end, and is modified with a TAMRA label at its 3' end. The latter modification not only increases signalling possibilities, but also prevents elongation by the polymerase. Samples S3 and S4 contained 10 ng of genomic DNA containing the target nucleic acid, which can be specifically amplified with the primers. Samples S9 and S10 serve as (negative) controls, as their SERRS signal will not decrease.

After adding Taq polymerase to the samples and performing a PCR reaction (3 step protocol, 35 cycles), the samples were diluted 100 times in water. SERRS detection was performed using a colloidal solution of EDTA reduced silver nanoparticles (prepared according to Heard et al. Journal of Colloid and Interface Science Vol 93 (1983), 535), aggregated using spermine (0.1 mM). The SERRS spectrum was measured using an excitation wavelength of 532 nm (Raman Systems, Inc.)

Results are shown in Fig. 7. As is expected, the highest SERRS signal is obtained from the negative controls, i.e. samples S9 and S10, where no target DNA has been amplified. These are indicated with a continuous and dotted line respectively. In samples S3 (dashed line, short dashes) and S4 (dashed line, long dashes), the PCR reaction was successful in amplifying the target DNA. The presence and amplification of the target DNA leads to a decrease in SERRS signal with a factor 1.7 (signal of the highest SERRS spectrum (without amplified target) divided by the signal of the sample (with amplified target) = 1.7).

### Example 4: SERRS signal in function of number of nucleotides coupled to SERRS dye

The SERRS signal has been measured as a function of number of nucleotides coupled to Rhodamine 6G. Rhodamine 6G dye was covalently coupled to 4, 6, 8 and 30 oligonucleotide probes. The concentration of R6G was 500 pM.

Briefly, the dye-labelled probe was added to a colloidal solution of EDTA reduced silver nanoparticles and the nanoparticles were aggregated using spermine (conditions as in example 1). The SERRS spectra were measured using an excitation wavelength of 532 nm (Raman Systems Inc). In Fig. 8 the background subtracted signal at 1650 cm⁻¹ is plotted as a function of number of nucleotides coupled to R6G. As the number of nucleotides decreases the SERRS signal decreases too. y-axis: SERRS signal in counts/second, x-axis: nr nucleotides.

### Example 5: Measuring SERRS spectra using single labelled SERRS probes

Comparison of SERRS spectra of non-amplified DNA with SERRS-labelled probes and amplified target DNA with SERRS labelled probes, while using a SERRS probe with a single label (see Fig. 9).

Four samples were tested, two did contain template DNA (S1, S2), two did not (S3, S4). To all samples 200 nM PCR primers were added. To each sample, 100 nM of a specific labelled 32-bases probe was added. The SERRS labelled probe carries an Atto520 label at its 5' end, and is modified with a phosphate group at its 3' end. The latter modification prevents elongation by the polymerase. Samples S1 and S2 contained 10 ng of genomic DNA containing the target nucleic acid, which can be specifically amplified with the primers. Samples S3 and S4 serve as (negative) controls, as their SERRS signal will not decrease.

After adding Taq polymerase to the samples and performing a PCR reaction (3 step protocol, 30 cycles), the samples were diluted 600 times in water. SERRS detection was performed using a colloidal solution of EDTA reduced silver nanoparticles aggregated using spermine (0.1 mM). The SERRS spectrum was measured using an excitation wavelength of 532 nm (Raman Systems, Inc.)

Results are shown in Fig. 9. For clarity spectra of sample S2 and S4 are shown only. As is expected the highest SERRS signal is obtained from the negative control, i.e. sample S4, where no target DNA has been amplified (solid line). In sample S2 (dashed line), the PCR reaction was successful in amplifying the target DNA. The presence and amplification of the target DNA leads to a decrease in SERRS signal with a factor 1.4 (signal of the highest SERRS spectrum (without amplified target) divided by the signal of the sample (with amplified target) = 1.4).

### Example 6: Comparison of SERRS spectra of a sample containing amplified non-target DNA and a "negative" sample (containing_primers but no template DNA)

Four samples were tested, two did contain template DNA (S 17, S 18), two did not (S 19, S20). To all samples 200 nM PCR primers were added. To each sample, 100 nM of a specific labelled 32-bases probe was added. This probe does not match the amplicon of S 17 and S18: the point here is to check whether the presence of DNA lowers the SERRS signal non-specifically. The SERRS labelled probe carries an Atto520 label at its 5' end, and is modified with a phosphate group at its 3' end. The latter modification prevents elongation by the polymerase. Samples S 17 and S18 contained 10 ng of genomic DNA containing the target nucleic acid, which can be specifically amplified with the primers. Samples S 19 and S20 serve as (negative) control, as their SERRS signal will not decrease.

After adding Taq polymerase to the samples and performing a PCR reaction (3 step protocol, 30 cycles), the samples were diluted 600 times in water. SERRS detection was performed using a colloidal solution of EDTA reduced silver nanoparticles aggregated using spermine (0.1 mM). The SERRS spectrum was measured using an excitation wavelength of 532 nm (Raman Systems, Inc.)

Results are shown in Fig. 10. For clarity, spectra of S 17 and S20 are shown only. As is expected, the SERRS signal is identical in all samples: the negative sample is indicated with a solid line, the positive with a dashed line, respectively. The PCR reaction was successful in amplifying the target DNA. The presence and amplification of the template DNA does not lead to a decrease in SERRS signal of the non-matching probe. This indicates that the method is specific.

## Claims

1. A method for detecting one or more target nucleic acids in a sample, comprising the steps of:
a) adding to the sample:
- one or more pairs of PCR primers each capable of amplifying one of the one or more target nucleic acids or a part thereof, and
- one or more SE(R)RS-labelled nucleic acid probes each capable of specifically hybridizing to one of the one or more target nucleic acids or part thereof that is amplified by the one or more PCR primer pairs;
b) performing a PCR reaction using a polymerase with 5'→3' exonuclease activity;
c) measuring the SE(R)RS signal of the sample as indication of the presence of the one or more target nucleic acids.

2. The method according to claim 1, wherein the polymerase is Taq polymerase.

3. The method according to claim 1, wherein each of the nucleic acid probes contains one SE(R)RS label.

4. The method according to claim 1, wherein the 3' end of the one or more nucleic acid probes is modified to prevent elongation of the probe during PCR.

5. The method according to claim 4, wherein the modification is by introducing an amine group, a phosphate group, a methyl group or a SE(R)RS label at the 3' end of the probe.

6. The method according to claim 1, wherein the SE(R)RS signal is monitored in real-time.

7. The method according to claim 1, wherein the SE(R)RS signal is measured as end-point measurement.

8. The method according to claim 7, further comprising comparing the SE(R)RS signal with that of a control containing no target nucleic acid, wherein a decrease in the SERRS signal compared to the control is indicative of the presence of target nucleic acid in the sample.

9. The method according to claim 6, further comprising comparing the SE(R)RS signal with the SE(R)RS signal of the sample measured after the labelled probes have been added, but prior to performing a PCR reaction, wherein a decrease in the SE(R)RS signal compared to the control is indicative of the presence of target nucleic acid in the sample.

10. The method according to claim 1, wherein the primers are capable of amplifying molecules related to the target nucleic acid sequence.

11. Use of the method according to claim 10 for detection of SNPs or particular alleles, or for sequencing.

12. The method according to claim 1, wherein the SE(R)RS signal is measured in the presence of a SERS-active substrate.

13. The method according to claim 12, wherein the SERS-active substrate comprises metal particles, in particular silver particles.

14. The method according to claim 12, wherein an aggregating agent is added to the colloidal SERS-active particles prior to measuring the SE(R)RS signal.

15. The method according to claim 14, wherein the aggregating agent is a polyamine, in particular spermine.
